## Europäisches Patentamt
## European Patent Office
## Office européen des brevets

(11) Numéro de publication: **0 041 019**
**B1**

(12) **FASCICULE DE BREVET EUROPEEN**

(45) Date de publication du fascicule du brevet: **31.10.84**

(21) Numéro de dépôt: **81400807.4**

(22) Date de dépôt: **21.05.81**

(51) Int. Cl.³: **C 07 C 103/52,** A 61 K 37/02, A 61 K 43/00, G 01 N 33/54

(54) **Nouveaux produits préparés à partir du facteur thymique sérique ou de ses analogues et dérivés, procédé de préparation de ces produits, ces produits pour utilisation comme médicaments, traceurs obtenus à partir de ces produits et utilisation de ces traceurs dans des procédés de dosage.**

(30) Priorité: **22.05.80 GB 8017027**
**22.09.80 GB 8030507**

(43) Date de publication de la demande:
**02.12.81 Bulletin 81/48**

(45) Mention de la délivrance du brevet:
**31.10.84 Bulletin 84/44**

(84) Etats contractants désignés:
**AT BE CH DE FR GB IT LI LU NL SE**

(56) Documents cités:

**COMPTES RENDUS DE L'ACADEMIE DES SCIENCES DE PARIS, série III, t. 292, Fasc. 13, 30 mars 1981, PARIS (FR) M. DARDENNE et al.: "Rôle du zinc et d'autres métaux dans l'activité biologique du FTS (thymuline)", pages 793-796 BIOLOGICAL ABSTRACTS, vol. 71, no. 7, page 4778, réf. 45560, Bioscience Information Service COLUMBUS, OHIO (US) R.K. CHANDRA et al.: "Serum thymic factor activity in deficiencies of calories, zinc, vitamin A and pyridoxine"**

(73) Titulaire: **INSTITUT NATIONAL DE LA SANTE ET DE LA RECHERCHE MEDICALE (INSERM)**
**101, rue de Tolbiac**
**F-75654 Paris Cedex 13 (FR)**

(72) Inventeur: **Bach, Jean-François**
**180, rue de Grenelle**
**F-75007 Paris (FR)**
Inventeur: **Pleau, Jean-Marie**
**8, rue Taucha**
**F91120 Palaiseau (FR)**
Inventeur: **Dardenne, Mireille**
**20, rue de Cronstadt**
**F-75016 Paris (FR)**
Inventeur: **Lefrancier, Pierre**
**46 Allée de la Mare l'Oiseau Chevry 2**
**F-91190 GIF-sur-Yvette (FR)**

(74) Mandataire: **Corre, Jacques Denis Paul et al**
**Cabinet Regimbeau 26, Avenue Kléber**
**F-75116 Paris (FR)**

Courier Press, Leamington Spa, England.

# 0 041 019

## Description

La présente invention concerne de nouveaux produits préparés à partir du Facteur Thymique Sérique (FTS) ou de ses analogues et dérivés, un procédé pour leur préparation, et ces produits pour utilisation comme médicaments.

Elle concerne également des traceurs radioactifs obtenus à partir de ces nouveaux produits et l'utilisation de ces traceurs dans un procédé de dosage radioimmunologique du FTS dans un échantillon.

Le FTS est un nonapeptide isolé à partir du sérum de porc et présent dans le thymus et le sérum normal de diverses espèces.

L'origine thymique du FTS, suggérée par la disparition du FTS du sérum après thymectomie, a été récemment directement démontrée par la localisation par immunofluorescence dans l'épithélium thymique d'anticorps purifiés produits contre le FTS de synthèse.

Le FTS possède de nombreuses activités biologiques *in vitro* et *in vivo* incluant en particulier l'induction de l'antigène thêta caractéristique de lymphocytes T chez la souris, sur des cellules thêta-négatives formant des rosettes obtenues à partir de la moelle osseuse et de la rate de souris thymectomisées.

Le FTS a été charactérisé par un test biologique, communément appelé "test de rosettes", fondé sur l'induction de la sensibilité à l'azathioprine de cellules formant des rosettes de la rate de souris thymectomisées. Le test de rosettes a été décrit par J. F. Bach et M. Dardenne dans Immunology, *26*, 353 (1973).

Ce test est fondé sur l'aptitude du FTS à rendre thêta-positives et sensibles à l'azathioprine les cellules thêta-négatives formant des rosettes, obtenues à partir de la rate de souris adultes thymectomisées. L'azathioprine est un analogue de la purine qui, comme le sérum anti-thêta, inhibe la formation de cellules formant des rosettes qui dépendent du thymus. Chez la souris normale, la formation de rosettes est inhibée par 1 $\mu$g/ml d'azathioprine, alors que chez la souris adulte thymectomisée, 50 à 100 $\mu$g/ml de ce composé sont nécessaires pour inhiber la formation de rosettes. Le FTS, ainsi que les extraits thymiques, confèrent aux cellules formant des rosettes de souris adultes thymectomisées, après incubation de 90 minutes à 37°C, une sensibilité à l'azathioprine identique à celle des cellules formant des rosettes de souris normales, ce qui permet de servir de base à un test biologique *in vitro* et *in vivo*, quantitatif et reproductible.

Selon le test de rosettes, on détermine l'activité thymique d'un échantillon (FTS ou extrait thymique ou échantillon de sérum) en le mettant en incubation dans un tube à hémolyse avec $3 \times 10^6$ cellules de la rate provenant de souris C 57/BL 6 adultes (fournies par le Centre d'Elevage des Animaux de Laboratoire du C.N.R.S. 45 Orléans, La Source) thymectomisées 10 à 20 jours plus tôt. La méthode de thymectomie est décrite par M. Dardenne, et J. F. Bach dans Immunology 26, 343 (1973) à la page 344, le contenu de cet article étant incorporé ici à titre de référence.

L'incubation est effectuée pendant 90 minutes à 37°C en présence d'azathioprine (Az) à une concentration de 10 $\mu$g/ml.

Cette concentration est intermédiaire entre la concentration minimale de Az inhibant 50% des cellules de la rate formant des rosettes (RFC) provenant de souris normales (1 $\mu$g/ml), et celle inhibant les cellules formant les rosettes provenant d'animaux thymectomisés (50 $\mu$g/ml). A la fin de l'incubation, $12 \times 10^6$ cellules rouges de sang de mouton (SRBC) sont ajoutées aux tubes contenant les cellules et l'échantillon. Les cellules et l'échantillon sont centrifugés pendant 6 minutes à 1400 tours/minute et remis en suspension doucement et avec précaution par rotation sur un agitateur rotatif (10 cm de diamètre) à petite vitesse (10 tours par minute). On compte les RFC dans un hématocymètre. En l'absence d'activité thymique, le nombre de RFC est de $1210/10^6$ cellules $\pm 120$ (écart type, SD). En présence d'activité thymique, la quantité de RFC est réduite à un niveau de 200 à $400/10^6$ cellules. En l'absence de Az, le FTS ne provoque pas d'inhibition des RFC.

Test de rosettes *in vitro*: l'activité thymique est définie comme la concentration minimale de l'échantillon qui induit l'inhibition de plus de 50% de cellules formant des rosettes de rate de souris adultes thymectomisées en présence de 10 $\mu$g/ml d'azathioprine.

*In vivo*: a) l'activité du sérum est exprimée sous forme de taux de facteur thymique (déterminé par le test de rosettes décrit ci-dessus) présent dans le sérum de souris adultes thymectomisées, recueilli 2 ou 4 heures après injection de 0,1 ng ou de 1 ng d'échantillon absorbé sur carboxyméthylcellulose ou injecté seul: l'activité du sérum est exprimée par la plus forte dilution du sérum permettant l'inhibition de 50% des cellules formant les rosettes.

b) l'activité des cellules de la rate est exprimée par la sensibilité à l'azathioprine de ces cellules de souris adultes thymectomisées, isolées 24 heures après injection de 0,1 à 1 ng de l'échantillon absorbé sur carboxyméthylcellulose ou injecté seul.

Le Demandeur a maintenant trouvé qu'il n'y avait pas toujours corrélation entre les activités mises en évidence dans les tests *in vitro* et *in vivo* et qu'il était possible d'obtenir des produits encore plus actifs que ceux obtenus à ce jour, particulièrement *in vivo*.

Le Demandeur a montré que, suivant les techniques employées, la synthèse chimique du FTS

2

pouvait éventuellement aboutir à un produit présentant une activité biologique *in vitro* et *in vivo* quantitativement inférieure (jusqu'à 100 et 1000 fois) à celle observée avec le FTS·d'origine naturelle.

D'autre part, le Demandeur a découvert que le traitement du FTS synthétique par un agent susceptible de chélater les métaux, tel qu'une résine chélatante donnait un produit ayant totalement perdu son activité biologique *in vitro* et *in vivo*.

Le Demandeur a pu mettre en évidence, au moyen d'une analyse en spectrométrie d'absorption atomique des différents lots de FTS, que les lots actifs contenaient de faibles quantités de métaux lourds, et en particulier du zinc, les lots inactifs en étant totalement dépourvus.

Le but essentiel de l'invention est de préparer de nouveaux produits à partir du FTS ou de ses analogues et dérivés afin d'obtenir des produits de qualité constante, pouvant être utilisés soit comme médicament, soit à la préparation de traceurs pour des procédés de dosage radioimmunologique.

L'invention concerne plus particulièrement de nouveaux produits caractérisés par le fait que le FTS ou l'un de ses analogues ou dérivés est lié à un métal susceptible de former un complexe de coordination IV ayant une configuration tétraédrique.

Le métal impliqué peut être un métal divalent comme le zinc ou le cuivre ou un métal trivalent comme l'aluminium, le zinc étant toutfois le métal préféré.

La quantité de métal présente dans les complexes de l'invention, telle que déterminée par spectrométrie atomique, doit être telle que le rapport en poids de métal au complexe soit supérieure à 1/200.

Les produits de l'invention présentent une activité thymique *in vivo* dans le test de rosettes indiqués précédemment, qui est multipliée par un facteur d'au moins 10 et même le plus souvent d'au moins 100 par rapport à l'activité du FTS de référence (FTS d'origine naturelle ou synthétique, tel qu'obtenu auparavant).

C'est ainsi que le produit de l'invention dans lequel le métal est le zinc, injecté à la dose de 0,1 ng par souris, présente une activité in vivo dans le test de rosettes 10 fois supérieure à celle obtenue pour le FTS de référence injecté à la dose de 1 ng par souris.

L'invention concerne également un procédé de préparation de nouveaux produits préparés à partir du FTS ou de ses analogues ou dérivés, consistant à traiter le FTS ou l'un de ses analogues ou dérivés avec un métal ou une substance protéique contenant un métal, ce métal étant susceptible de se lier au FTS ou son analogue ou dérivé et de former un complexe de coordination IV de configuration tétraédrique.

Dans un mode de réalisation préféré de l'invention, le FTS ou l'un de ses analogues ou dérivés est mis en contact avec le métal ou l'un de ses sels, à un pH compris entre 7 et 9, pendant 10 à 30 minutes, et à la température ambiante, par exemple de 20 à 37°C.

Dans ce mode de réalisation, on utilise avantageusement une proportion de 1 mole de FTS ou d'analogue ou dérivé de FTS pour 0,1 à 10 moles du métal ou d'un de ses sels, de préférence 1 mole du métal ou d'un de ses sels.

Dans un autre mode de réalisation du procédé de l'invention, on prépare les nouveaux produits par traitement du FTS ou l'un de ses analogues ou dérivés avec une substance protéique contenant un métal.

Cette substance protéique contenant un métal peut être par exemple, un fraction de sérum ou une fraction d'un extrait thymique.

A titre d'exemple, on peut utiliser comme substance protéique contenant un métal, une fraction de la Thymosine Fraction 5, que l'on appellera ci-après "fraction activatrice" en raison de ses propriétés activatrices à l'égard du FTS ou des ses analogues et dérivés.

A. L. Goldstein et coll. ont isolé en 1965 à partir de thymus de veau, une préparation peu purifiée qu'ils ont appelée Thymosine Fraction 5 et qui est un mélange de nombreux polypeptides ayant des activités biologiques variées.

Cette préparation a été utilisée chez l'homme dans des essais cliniques de Phases 1 et 2 avec des résultats non encore concluants, mais prometteurs.

Plus récemment, A. L. Goldstein a publié la séquence de certains peptides contenus dans la fraction 5, à savoir les thymosines $\alpha_1$, $\beta_1$,... mais aucun de ces peptides ne s'est révélé avoir d'homologie de structure avec le FTS.

Des études préalables effectuées par le Demandeur ont montré que la Thymosine fraction 5 contenait une ou des substances actives dans le test des rosettes cité plus haut, et ayant des propriétés physiques ou chimiques comparables à celles du FTS (poids moléculaire, charge électrique, composition en acides aminés).

La fraction activatrice mentionnée ci-dessus est préparée par fractionnement de Thymosine Fraction 5 dans une colonne de G-100 (Pharmacia 0,9 cm × 100 cm) en tampon phosphate 0,2 M pH 7,3 afin d'éliminer la (les) molécule (s) semblable (s) au FTS, les fractions éluées avec des volumes d'élution compris entre 15 et 22 ml étant seules conservées.

Cette fraction contient des molécules de poids moléculaire compris entre 7 500 et 12 500 (traceurs utilisés: IGG, albumine bovine, Cytochrome C et FTS tritié).

Cette fraction est sensible à la chaleur: son activité est détruite par chauffage à 60°C en moins de 15 minutes et à 100°C en moins de 5 minutes.

Cette fraction, qui est elle-même dépourvue d'activité dans le test des rosettes, présente des propriétés activatrices à l'égard du FTS ou de ses analogues et dérivés en ce sens qu'elle permet d'augmenter d'un facteur d'environ $10^3$ l'activité biologique de ces derniers.

Le procédé de préparation des nouveaux produits à partir de la fraction activatrice provenant de la Thymosine Fraction 5 est caractérisé par le fait que l'on fait incuber le FTS ou l'un de ses analogues ou dérivés avec cette fraction activatrice, on fractionne le mélange obtenu après incubation, et on sélectionne la fraction la plus active dans le test des rosettes.

Dans ce procédé de préparation selon l'invention, la température d'incubation est comprise entre 30 et 40°C, la durée de l'incubation est comprise entre 10 minutes et 1 heure et l'incubation est conduite à un pH compris entre 6 et 8.

Les conditions spécialement préférentielles consistent à conduire l'incubation à une température d'environ 37°C pendant 20 minutes à pH 7,3.

Quel que soit le procédé de préparation utilisé (traitement avec un métal ou sel de métal, ou traitement avec une substance protéïque contenant un métal) il est préférable d'éliminer au préalable toute trace de métal éventuellement présente dans la préparation du FTS ou d'un de ses analogues ou dérivés au moyen d'un agent de chélation capable de chélater les métaux, comme par exemple une chromatographie sur une colonne de résin chélatante du type "Chelex 100®". Ceci permet d'obtenir un produit chimiquement homogène qui est biologiquement inactif et qui, après traitement avec le métal ou l'un de ses sels, donnera un produit homogène avec des résultats reproductibles, et donc acceptable pour une utilisation pharmaceutique.

Le FTS ou les analogues ou dérivés du FTS utilisées dans l'invention sont ceux qui ont été synthétisés en phase liquide selon la méthodologie décrite par Bricas, E., Martinez, J., Blanot, D., Auger, G., Dardenne, M., Pleau, J. M. et Bach, J. F. 5th Intern. Peptide Symposium, La Jolla, Californie, E.U.A. Juin 1977, et ceux revendiqués dans la demande de brevet français 77 15963 du 25 Mai 1977 et son addition 78 11870 du 21 Avril 1978.

Le FTS est un nonapeptide de séquence p Glu-ALa-Lys-Ser-Gln-Gly-Gly-Ser-Asn et les analogues et dérivés du FTS dont il est fait référence ici dérivent de cette séquence de la façon indiquée dans la demande de brevet français et l'addition indiqués ci-dessus.

La Thymosine Fraction 5 utilisée pour la préparation de la fraction activatrice de l'invention a été obtenue selon la méthode de A. L. Goldstein (Goldstein A. L. Salter, F. D and White A., 1966, Proc. Nat. Acad. Sci. USA, *56*, 1010—1017, et Goldstein, A. L., Guha, A., Zatz, M. M., Hardy, M., White, A., Proc. Nat. Acad. Sci. USA, *69*, 1800—1803).

D'autres caractéristiques de l'invention seront maintenant décrits en référence aux exemples suivants.

Exemple 1

Préparation de la fraction activatrice

La Thymosine Fraction 5, préparée selon la méthode ci-dessus, a, dans un premier temps, été fractionnée dans une colonne de G-100 (Pharmacia 0,9 cm×100 cm) en tampon phosphate 0,2 M pH 7,3 afin d'éliminer la (les) molécule (s) citée (s) plus haut et semblable (s) ou identique (s) au FTS.

Les fractions éluées avec des volumes d'élution compris entre 15 et 22 ml sont seules conservées et utilisées pour les expériences décrites. Ces fractions qui seront désignées désormais comme "fractions activatrices" contiennent des molécules de poids moléculaire compris entre 7500 et 12 500 et sont dépourvues d'activité dans le test des rosettes.

Les quantités de fractions activatrices utilisées seront exprimées dans ce qui suit, en équivalent Fraction 5 (EqF 5) c'est-à-dire en équivalent de la quantité de Fraction 5 utilisée pour la préparer.

Exemple 2

Préparation d'un produit activé

Un mg de FTS répondant à la formule p Glu-Ala-Lys-Ser-Gln-Gly-Gly-Ser-Asn, synthétisé en phase liquide selon la méthodologie indiquée ci-dessus, est incubé avec 50 mg (EqF 5) de la fraction activatrice de l'Exemple 1, dans un tube en matière plastique pendant 20 minutes à 37°C en tampon phosphate 0,2 M pH 7,3.

Au terme de cette incubation, le mélange est appliqué sur une colonne de Sephadex G-25® et la fraction active, détectée par le test des rosettes, est récupérée dans les fractions ayant un volume d'élution compris entre 1,8 et 2,2 Vo.

Cette fraction est dessalée par filtration à sec sur une membrane Amicon UM 2® puis reprise dans un tampon phosphate 0,02 M pH 6,1 et appliquée sur une colonne de carboxyméthylcellulose (Whatman), équilibrée par le même tampon phosphate.

Un gradient de NaCl est appliqué après passage de 50 ml du tampon initial. Les fractions actives dans le test des rosettes sont retrouvées dans les fractions contenant 0,12 M de NaCl.

Les fractions obtenues au terme de l'incubation avec la fraction activatrice sont $10^2$ à $10^3$ plus actives que la préparation initiale de FTS.

Ces fractions actives sont ensuite dessalées par filtration à sec sur membrane Amicon UM 2® puis soumises à une analyse d'acides aminés.

Les résultats de cette analyse montrent que la fraction ainsi récupérée a une activité biologique élevée et a une composition en acides aminés compatible avec celle du FTS initial.

Il en résulte que l'incubation du FTS avec la fraction activatrice de l'invention, issue d'un extrait thymique, mais dépourvue elle-même d'activité biologique dans le test des rosettes, augmente d'un facteur $10^3$ l'activité biologique de FTS de synthèse.

L'analyse d'acides aminés et le comportement sur Sephadex G-25® et carboxyméthylcellulose de la préparation "activée" confirment qu'il s'agit du FTS de synthèse, lui-même,, activé.

En présence de la fraction activatrice (distincte du FTS), le FTS est transformé au terme de 20 minutes à 37°C et cette transformation augmente de façon très significative son activité biologique.

Cette transformation implique, comme dans le cas du traitement par un métal ou un sel de métal, une fixation d'un métal sur le produit de départ.

On a pu démontrer que ce métal provient effectivement de la fraction activatrice. En effet, ces fractions activatrices perdent, après passage sur une agent de chélation des métaux (par exemple Chelex 100®), leur pouvoir d'activer le FTS ou ses analogues et dérivés.

Exemple 3
Activation par un metal
Materiel et methodes

1. Re'actifs

Le FTS de synthèse a été préparé selon la méthode indiquée ci-dessus. Le FTS marqué au tritium a été obtenu par tritiation d'un analogue du FTS contenant une lysine possédant une liaison acétylénique (Sasaki A. N., Morgat J. L., et E. Bricas 16° Symposium Européen des Peptides. Elsinger, Danemark, Septembre 1980 K. Brungeldt ed. p. 224).

Le zinc a été utilisé sous forme de chlorure éventuellement radiomarqué ($^{65}ZnCl_2$).

2. Chromatographies

Le traitement par Chelex 100® (Biorad Lab.) a été réalisé en mélangeant 0,2 ml de la solution contenant le produit étudié avec 0,2 ml de suspension de Chelex® à 50 mg/ml. La chromatographie sur Biogel P-2® a été faite en utilisant des colonnes de plastique (60×0,9 cm).

3. Essai des rosettes

Les cellules spléniques de souris C57BL/6 thymectomisées à l'âge de six semaines sont résistantes à l'azathioprine (10 $\mu$g/ml). Les extraits thymiques ou le FTS ou les sérums qui le contiennent leur confèrent la sensibilité à l'azathioprine. Les résultats de l'essai sont exprimés par la concentration minimale de FTS ou la dilution maximale de sérum inhibitrice.

II Resultats
1. Perte de l'activité biologique du FTS en présence d'un agent chelateur de métaux

Une solution aqueuse de FTS de synthèse est mise en contact avec la résine Chelex 100®. Le FTS non retenu par la résine est testé dans l'essai des rosettes. Le Tableau I ci-dessous montre que le FTS a perdu après ce traitement l'essentiel de son activité in vitro et in vivo. Des résultats analogues sont observés avec du FTS naturel (ultrafiltrat de sérum normal de souris) traité dans les mêmes conditions.

2. Réapparition de l'activité biologique du FTS prétraité par passage sur Chelex 100®, après addition de zinc ou d'autres métaux

L'addition de 10 ng de Cl$_2$Zn à 100 ng de FTS (synthétique ou naturel) prétraité par passage sur Chelex 100®, suivie d'une incubation de 10 minutes à 20°C, entraîne non seulement la restauration complète de l'activité biologique in vivo et in vitro, mais même un important accroissement de cette activité par rapport à celle observée initialement avant tout traitement (Tableau 1). Le chlorure de zinc est inactif par lui-même dans l'essai des rosettes in vitro et in vivo. L'activation du FTS est optimale à pH neutre (ou légèrement alcalin). La nécessité d'un contact entre le zinc et le FTS est démontrée par l'absence de réapparition de l'activité biologique observée dans les expériences in vivo lorsque le FTS passé sur Chelex 100® et le chlorure de zinc sont injectés à une minute d'intervalle. D'autres métaux que le zinc peuvent activer le FTS: l'aluminium (aussi actif que le zinc), le cuivre, le manganèse, le chrome, le fer et le nickel (nettement moins actifs que le zinc) alors que l'indium, le thallium, le cadmium, le plomb, le cobalt et l'étain restent sand effet.

3. Démonstration directed de la fixation du zinc sur la molécule de FTS

Le FTS tritié est élué à l'eau distillée d'une colonne de Biogel P-2® en un seul pic (fractions 10—14, Ve/Vo:1,3) qui recouvre l'activité biologique dont le niveau est plus élevé dans les toutes premières fractions (10—12). Appliqué sur la même colonne, le $^{65}ZnCl_2$ est élué avec un Ve/Vo de 2,2 (fractions 19—22). Lorsqu'un mélange de 0,1 $\mu$g de FTS tritié (préalablement passé sur Chelex 100®) et de 10 à 30 ng de $^{65}ZnCl_2$ est appliqué sur la colonne de Biogel P-2®, la radio activité correspondant à $^{65}Zn$ apparaît en deux pics: le premier, le plus important, correspond très précisément à l'activité biologique et à la première moitié du pic de radioactivé du FTS tritié (Fractions 9—12), le second à celui du zinc libre (fractions 19 à 23). Ce résultat montre qu'un pourcentage d'environ 10% de zinc s'est fixée sur le FTS.

III. Discussion

Ces expériences montrent que le FTS perd son activité après passage sur une résine chelatant les métaux, et la retrouve intégralement après addition de divers sels métalliques tels que ceux du zinc ou de l'aluminium et partiellement après addition de certains autres. Le zinc se fixe sur le FTS avec une constante d'affinité suffisante pour permettre une séparation sur Biogel P-2® du zinc libre. Ainsi le FTS n'est actif qu'après avoir capté certains métaux et plus particulièrement le zinc.

La présence de l'activité biologique et de la fixation du zinc dans les toutes premières fractions du pic de FTS élué sur Biogel P-2® indique l'existence de deux formes de FTS. Seule la première d'entre elles ayant lié le zinc est biologiquement active dans l'essai des rosettes. D'autres résultats indiquent que ces deux formes diffèrent également par leur antigénicité.

Le tableau I rassemble les résultats des tests in vivo et in vitro et montre l'augmentation de l'activité biologique des produits activés de l'invention obtenus par traitement du FTS soit avec un mètal, soit avec la fraction activatrice issue de la Thymosine Fraction 5.

**0 041 019**

TABLEAU I

| | Activité in vitro: Concentration minimale inhibitrice | Activité in vivo: Dilution du sérum inhibitrice 30 min après l'administration de 1 ng de FTS |
|---|---|---|
| FTS synthétique | $5 . 10^{-6}$ ng/ml | 1/80 000 |
| FTS synthétique passé sur Chelex 100® | 1 ng/ml | 1/256 |
| FTS synthétique passé sur Chelex 100®+Chlorure de zinc | $2 . 10^{-7}$ ng/ml | $1/10^6$ |
| Chlorure de zinc seul | pas d'activité | 1/4 |
| FTS synthétique | $5 . 10^{-6}$ ng/ml | 1/80 000 |
| FTS synthétique passé sur Chelex 100®+fraction activatrice | $3 . 10^{-9}$ ng/ml | $1/10^6$ |
| FTS synthétique passé sur Chelex 100®+fraction activatrice passée sur Chelex 100® | 1 ng/ml | <1/100 |
| Sérum normal de souris (ultrafiltrat) | | 1/128 |
| Sérum normal passé sur Chelex 100® | | 1/4 |
| Sérum normal passé sur Chelex 100®+chlorure de zinc | | 1/128 |
| Sérum normal passé sur Chelex 100®+fraction activatrice | | 1/128 |
| Sérum normal passé sur Chelex 100®+fraction activatrice passée sur Chelex 100® | | 1/4 |
| Sérum de souris thymectomisées | | 1/4 |
| Sérum de souris thymectomisées+chlorure de zinc | | 1/4 |
| Sérum de souris thymectomisées+fraction activitrice | | 1/4 |

Le tableau I montre que le FTS prétraité par passage sur Chelex 100® retrouve ensuite une activité biologique largement accrue in vitro et encore plus in vivo après traitement par un métal ou par la fraction activatrice, ce métal et cette fraction activatrice étant eux-mêmes dépourvus d'activité biologique dans le test des rosettes.

7

Il ressort également du tableau I que certains produits de l'invention se sont révélés actifs à des concentrations inférieures à $10^{-9}$ ng/ml.

Pour les tests in vivo,

les dérivés activés de l'invention ont été injectés à des souris thymectomisées à l'âge de six semaines et utilisées huit semaines après la thymectomie. Ils entraînent l'apparition à partir de la 30ème minute dans le sérum d'une activité biologique 10 à 100 fois supérieure à celle obtenue avec le FTS natural convérant à des cellules spléniques formant des rosettes la sensibilité à l'azathioprine.

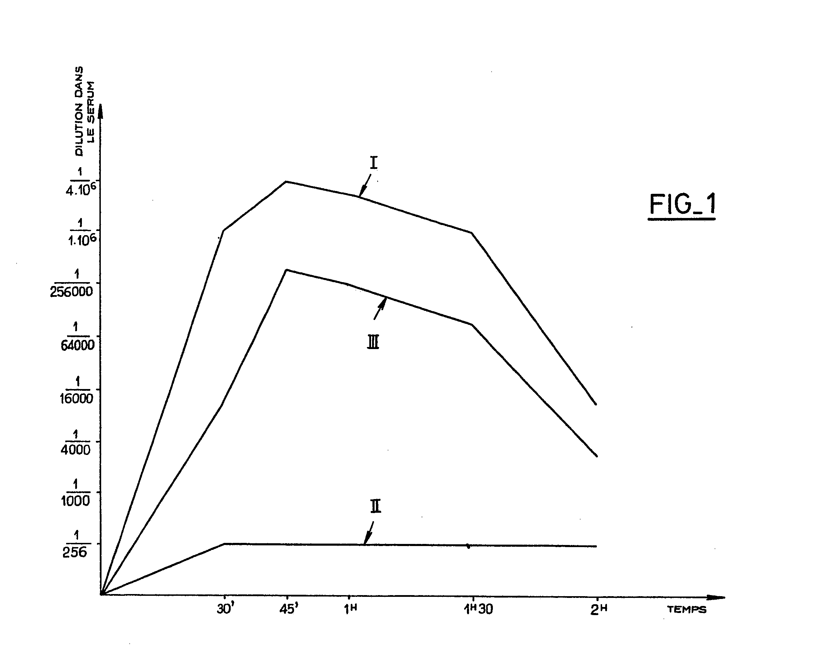

Cette augmentation de l'activité biologique est bien montrée sur la figure 1 qui compare l'activité biologique du FTS, passé sur une résine chélatante puis traité par le sulfate de zinc a pH 7 et injecté à la dose de 0,1 ng par souris (courbe I), à l'activité du FTS uniquement passé sur la résine puis injecté à la dose de 1 ng par souris (courbe II) et à l'activité du FTS de référence injecté à la dose de 1 ng par souris (courbe III).

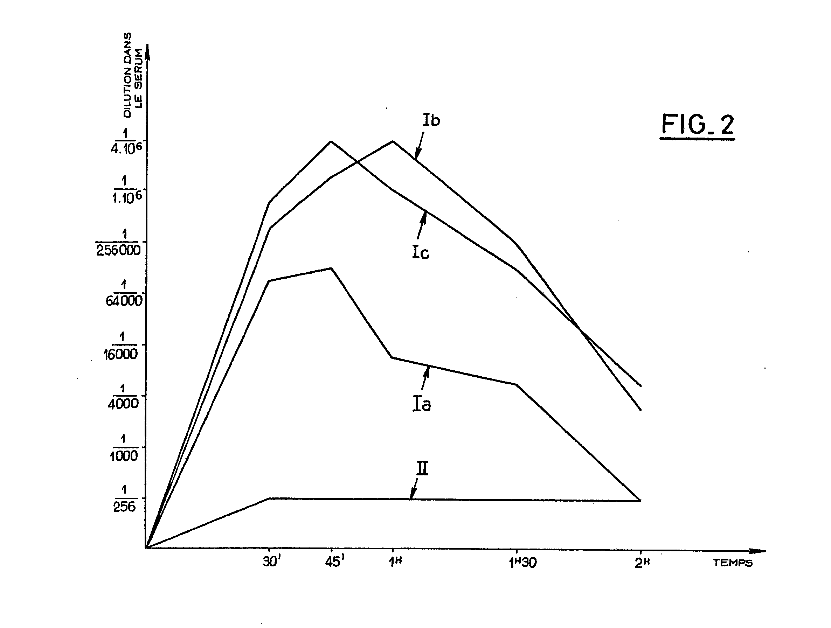

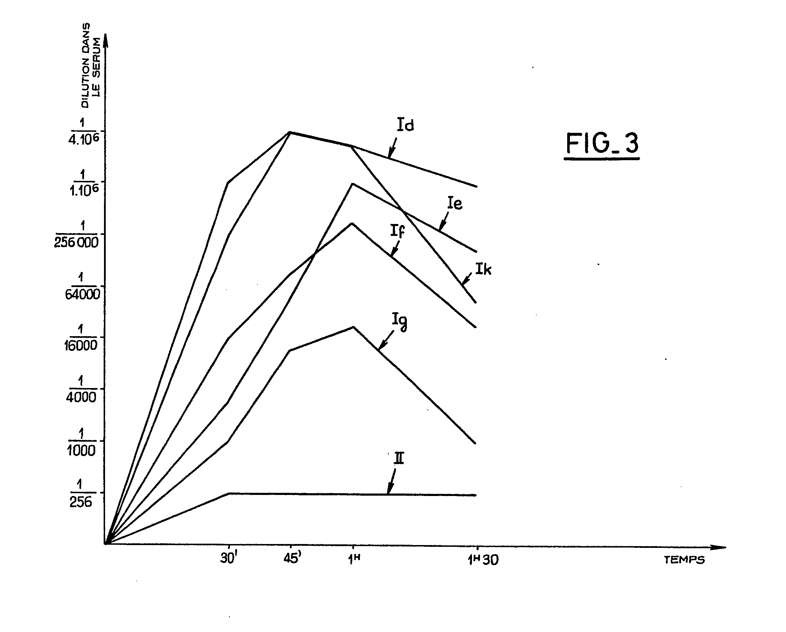

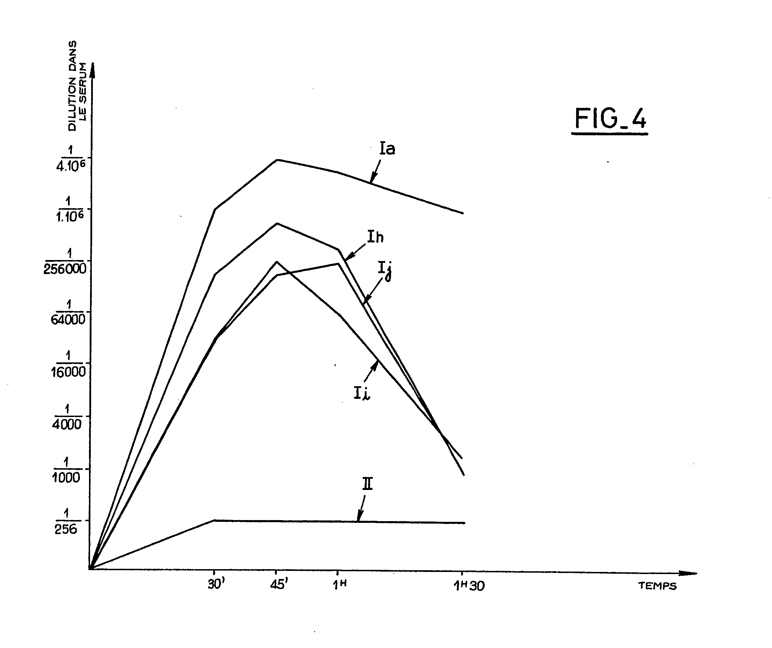

Les courbes de la figure 1, de même que celles des figures 2 à 6, expriment la variation de l'activité du produit injecté dans le sérum, exprimée par la dilution active de sérum en fonction du temps écoulé après l'injection. Les coubres de la figure 1 comparent l'activité du FTS de référence injecté à la souris l'activité du FTS traité par une résine chélante et injecté à la souris et l'activité d'un produit selon l'invention injecté à la souris, ce dernier étant beaucoup plus actif.

La figure 2 montre l'activation obtenue in vivo par les FTS passé sur résine chélatante puis traité par le sulfate de zinc pour des rapports pondéraux Zn/FTS différents [0,01 (courbe Ia); 0,1 (courbe Ib); 1 (courbe Ic)] et injecté à la souris par comparaison avec le FTS uniquement passé sur résine chélatante, et injecté à la souris (courbe II).

La meilleure activation est obtenue pour les rapports pondéraux de 0,1 à 10.

Les figures 3 et 4 comparent les activités obtenues in vivo par les FTS passé sur résine chélatante puis traité par différents métaux, à savoir les sels métalliques suivants:

Sulfate de zinc (courbe Id)
Sulfate de cuivre (courbe Ie)
Sulfate ferreux (courbe If)
Chlorure de cobalt (courbe Ig)
Chlorure de nickel (courbe Ih)
Chlorure de chrome (courbe Ii)
Chlorure de manganèse (courbe Ij)
Nitrate d'aluminium (courbe Ik)

et injecte à la souris, à l'activité obtenue avec le FTS uniquement passé sur résine chélatante et injecté à la souris (courbe II).

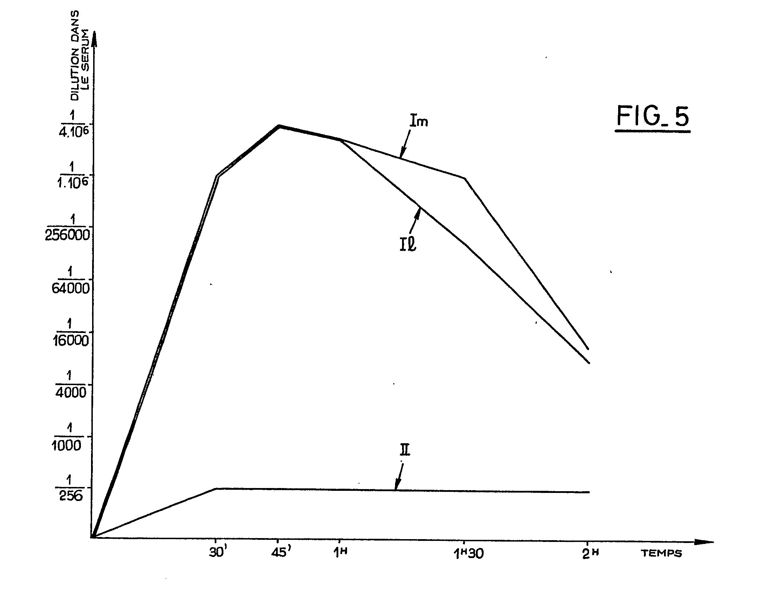

La figure 5 montre l'activité obtenue in vivo par le FTS passé sur résine chélatante puis traité par le sulfate de zinc pendant soit 10 minutes à 20°C (courbe Il) soit 10 minutes à 37°C (courbe Im), et injecté à la souris par comparaison au FTS uniquement passé sur résine chélatante et injecté à la souris. Cette figure montre qu'il n'y a pas de différences d'activation significatives entre le traitement effectué à 20°C et celui effectué à 37°C.

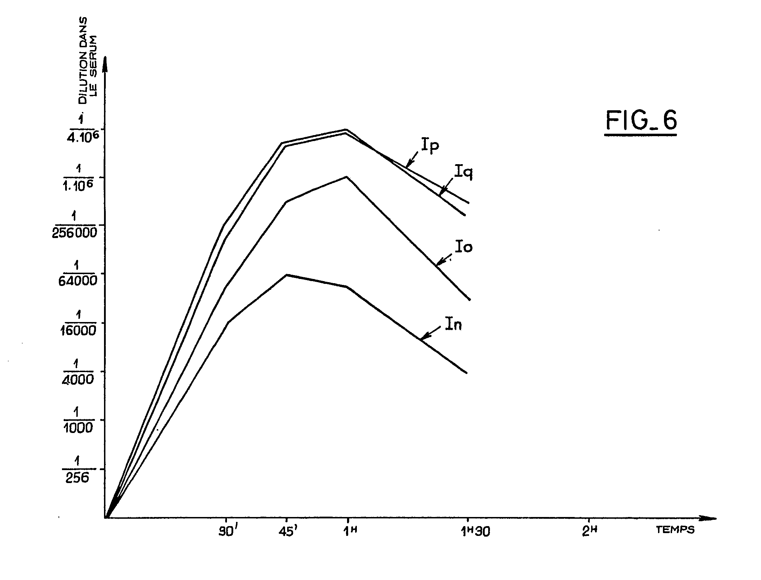

La figure 6 montre l'activité obtenue in vivo par le FTS passé sur résine chélatante puis traité par le zinc pour différentes doses d'injection à la souris: 0,001 ng (courbe In), 0,01 ng (courbe Io), 0,1 ng (courbe Ip) et 1 ng (courbe Iq).

Parallèlement, le traitement in vivo de souris thymectomisées par les produits de l'invention restaure totalement la sensibilité à l'azathioprine et au sérum anti-thêta des cellules formant les rosettes de la rate de ces souris, comme le montrent les résultats du tableau II ci-après.

8

TABLEAU II

Sensibilite a l'azathioprine de la rate de souris thymectomisees (Tx) injectee 24 h avant le test par 0,1
ou 1 ng de FTS de reference ou de produits de l'invention

| Traitement | Sensibilité de la rate à l'azathioprine |
|---|---|
| Rate normale non traitée | $0,7\ \gamma$ |
| Rate Tx non traitée | $25\ \gamma$ |
| Rate Tx traitée par 1 ng de FTS passé sur Chelex 100® | $25\ \gamma$ |
| Rate Tx traitée par 1 ng de FTS de référence | $0,7\ \gamma$ |
| Rate Tx traitée par 0,1 mg de FTS passé sur Chelex 100® et tràité par 0,1 ng de $ZnSO_4$ | $0,7\ \gamma$ |
| Rate Tx traitée par 0,01 ng de FTS passé sur Chelex 100® et traité par 0,1 ng de $ZnSO_4$ | $0,7\ \gamma$ |
| Rate Tx traitée par 0,1 ng de FTS passé sur Chelex 100®+fraction activatrice | $0,7\ \gamma$ |
| Rate Tx traitée par 0,1 ng de FTS passé sur Chelex 100®+fraction activatrice passée sur Chelex 100® | $25\ \gamma$ |

Ces expériences ont été répétées à différentes doses de produits: 1 à 100 pg, ce qui a permis de démontrer une relation dose-effet et montrer l'absence de toxicité aiguë, que le produit soit injecté seul (dans du sérum physiologique) ou absorbé sur de la carboxyméthyl cellulose.

Ces résultats montrent qu'*in vivo* une activation importante est obtenue par le zinc et à un moindre degré par d'autres métaux.

Les produits activés de l'invention présentent les mêmes propriétés pharmacologiques et les mêmes applications thérapeutiques que le FTS ou les analogues ou dérivés du FTS dont ils dérivent.

Il a déjà été indiqué dans les publications citées plus haut et relatives aux analogues et dérivés du FTS que ces composées peuvent présenter soit la même activité thymique que le FTS soit une activité inhibitrice ou antagoniste de cette activité thymique.

Les composés actifs dans le test des rosettes présentent tous la même activité thymique que le FTS, alors que certains composés inactifs dans le test des rosettes possédent une action inhibitrice ou antagoniste de l'activité thymique.

Dans tous les cas ces dérivés sont utiles comme médicaments pour le traitement de maladies consécutives à un déséquilibre des lymphocytes T, notamment des maladies auto-immunes correspondant à un effondrement des lymphocytes suppresseurs; des maladies virales correspondant à une biasse des lymphocytes "helpers"; des maladies tumorales dues à un déficit en lymphocytes "killers".

Le tableau III ci-dessous montre l'effet du traitement par le zinc de certains analogues et dérivés du FTS vis-à-vis de leur activité biologique.

TABLEAU III

| Peptide | Activité biologique (test des rosettes) | | Greffes de Peau | Activité en présence de zinc | | Inhibiteur | Liaison aux anticorps | Récepteur |
| | In vitro | In vivo | | In vitro | In vivo | | | |
|---|---|---|---|---|---|---|---|---|
| 36 | — | — | — | — | — | — | — | — |
| 37 | — | — | ND | | + | — | + | — |
| 38 | ± | ± | ND | | + | — | + | ± |
| 42 | — | — | ND | | | + | — | + |
| 18 | + | + | ND | | | — | + | + |
| 47 | — | — | ND | | ++ | — | + | — |
| 45 | — | — | ND | + | ++ | — | + | — |
| 53 | — | — | ND | — | — | + | — | + |
| 59 | — | — | ND | — | — | + | + | + |
| 60 | — | — | ND | | | | ± | ± |
| 34 | + | + | ND | | | | + | + |
| 61 | + | + | ND | | | — | ± | + |
| 62 | + | +R | +R | | | | + | — |
| 63 | — | — | ND | | + | — | + | + |
| 64 | + | + | ND | | | | + | + |
| 66 | — | — | — | — | — | — | — | — |
| 71 | — | — | ND | — | — | | — | — |
| 77 | — | — | ND | — | — | — | + | ± |
| 79 | — | — | ND | — | — | + | — | + |
| 82 | — | — | ND | — | — | — | + | — |
| 83 | — | — | ND | — | — | — | + | — |
| 84 | — | — | ND | + | ++ | — | + | — |
| 85 | — | — | ND | — | — | — | + | — |
| 86 | — | — | ND | — | — | + | + | + |
| 88 | — | — | ND | — | — | — | ± | — |
| 89 | + | +R | R | — | ++ | — | + | + |
| 90 | + | +R | R | — | — | — | — | + |
| 91 | ± | ±R | ND | — | — | — | + | + |
| 93 | — | — | ND | — | — | + | — | + |
| 96 | — | — | ND | + | + | + | — | + |

TABLEAU III (suite)

| Peptide | Activité biologique (test des rosettes) | | Greffes de Peau | Activité en présence de zinc | | Inhibiteur | Liaison aux anticorps | Récepteur |
| | In vitro | In vivo | | In vitro | In vivo | | | |
|---|---|---|---|---|---|---|---|---|
| 97 | — | — | ND | — | — | + | — | + |
| 105 | — | + | ND | — | — | — | + | + |
| 106 | + | + | ND | — | — | — | + | + |
| 107 | + | + | ND | — | — | — | + | + |
| 109 | + | + | ND | | — | | — | + | + |
| 111 | — | — | ND | — | — | — | + | — |
| 112 | + | ++R | ++R | — | — | — | + | + |
| 113 | — | — | | | | — | — | — |

Il est important de remarquer que certains peptides (peptides n° 37, 38, 47, 45, 60, 63, 84, 96) qui sont inactifs ou peu actifs dans le test des rosettes deviennent actifs après traitement par le zinc.

Dans le tableau III, le symbole ND signifie non déterminé, le symbole R signifie que le composé correspondant à une activité retard, c'est-à-dire qui se prolonge dans le temps.

D'autre part les numéros de peptides correspondent aux peptides suivants:

Ser-Gln-Gly-Gly-Ser-Asn (36)
Lys-Ser-Gln-Gly-Gly-Ser-Asn (37)
Ala-Lys-Ser-Gln-Gly-Gly-Ser-Asn (38)
PyroGlu-Ala-Lys-Ser-Gln-Gly-Gly-Ser (42)
Gln-Ala-Lys-Ser-Gln-Gly-Gly-Ser-Asn (18)
PyroGlu-D-Ala-Lys-Ser-Gln-Gly-Gly-Ser-Asn (47)
D-Ala-Lys-Ser-Gln-Gly-Gly-Ser-Asn (45)
PyroGlu-Ala-Lys-Ser-Gln-Gly-Gly-Ser-Asp (53)
PyroGlu-Ala-Lys-Ser-Gln-Gly-Gly-Ala-Asn (59)
PyroGlu-Ala-Lys-Ala-Gln-Gly-Gly-Ser-Asn (60)
Z Gln-Ala-Lys-Ser-Gln-Gly-Gly-Ser-Asn (34)
PyroGlu-Ala-D-Lys-Ser-Gln-Gly-Gly-Ser-Asn (61)
PyroGlu-Ala-Lys(N$^6$-acétyl)-Ser-Gln-Gly-Gly-Ser-Asn (62)
PyroGlu-Ala-Orn-Ser- Gln-Gly-Gly-Ser-Asn (63)
PyroGlu-Ala-Lys-(N-methyl)Ser-Gln-Gly-Gly-Ser-Asn (64)
PyroGlu-Ala-Lys-D-Ser-Gln-Gly-Gly-Ser-Asn (66)
PyroGlu-Ala-Lys-Ser-D-Gln-Gly-Gly-Ser-Asn (71)
PyroGlu-Ala(2,6-diaminohexynoyl)-Ser-Gln-Gly-Gly-Ser-Asn (77)
PyroGlu-Ala-Lys-Ser-Gln-Gly-Gly-Ser-Gln (79)
Lys(N$^6$ acétyl)-Ser-Gln-Gly-Gly-Ser-Asn (82)
D-Lys-Ser-Gln-Gly-Gly-Ser-Asn (83)
Orn-Ser-Gln-Gly-Gly-Ser-Asn (84)
Nx Z-Lys-Ser-Gln-Gly-Gly-Ser-Asn (85)
PyroGlu-Ala-Lys-Ser-Gln-Gly-Gly-Ser-$\beta$-Ala-NH$_2$ (86)
Lys-Ser-Gln-D-Ala-Gly-Ser-Asn (88)
PyroGlu-Ala-Lys-Ser-Gln-D-Ala-Gly-Ser-Asn (89)
PyroGlu-Ala-Lys-Ser-Gln-Gly-Gly-Ser-D-Asn (90)
PyroGlu-Ala-Hep-Ser-Gln-Gly-Gly-Ser-Asn (91)
PyroGlu-Ala-Lys-Ser-Gln-Gly-Gly-Ser-Asn-NH$_2$ (93)
PyroGlu-Ala-Lys-Ser-Asn-Gly-Gly-Ser-Asn (96)
PyroGlu-Ala-Lys-Ser-Nva-Gly-Gly-Ser-Asn (97)
Z-Ala-Lys Ser-Gln-Gly-Ser-Asn (105)
D-PyroGlu-Ala-Lys-Ser-Gln-Gly-Gly-Ser-Asn (106)
D-Gln-Ala-Lys-Ser-Gln-Gly-Gly-Ser-Asn (107)
Pro-Ala-Lys-Ser-Gln-Gly-Gly-Ser-Asn (109)
PyroGlu-Ala-Arg-Ser-Gln-Gly-Gly-Ser-Asn (111)
PyroGlu-Ala-Har-Ser-Gly-Gly-Gly-Ser-Asn (112)
PyroGlu-Ala-Lys(N$^6$-acétyl)-Ser-Gln-D-Ala-Gly-Ser-Asn (113)

**0 041 019**

Dans les peptides ci-dessus, les acides aminés sont désignés sous leurs abréviations habituelles. De plus Z désigne le groupe benzyloxycarbonyle.

Comme indiqué plus haut, les produits activés de l'invention sont utiles dans le traitement de maladies consécutives à un déséquilibre des lymphocytes T, notamment correspondant à:

— un effondrement des lymphocytes T suppresseurs: maladies auto-immunes telles que Lupus, en particulier Lupus érythémateux, sclérose en plaque, polyarthrites rhumatoïdes;
— une baisse des lymphocytes "helpers": infections bactériennes et virales, en particulier herpès récidivant, zona, varicelle;
— un déficit en lymphocytes "killers": maladies tumorales, en particulier cancers naso-pharyngiens, cancer épidermoïde du poumon, cancer infiltrant de la vessie, mélanomes, néoplasies.

Les produits de la présente invention peuvent être administrés par voie intraveineuse ou intramusculaire, par voie de suppositoires, par voie d'aérosols ou par voie orale sous forme protégée, des véhicules appropriés qui peuvent être utilisés dans la composition comprennent, par exemple, des liquides stériles comme l'eau ou un soluté physiologique ou des substances susceptibles de conférer un effet retard ou encore des préparations permettant l'administration par voie de suppositoires, par voie d'aérosols ou par voie sublinguale. En plus d'un véhicule, les présentes compositions peuvent aussi comprendre d'autres ingrédients tels que des stabilisateurs, des antioxydants, des agents de suspension ou des agents de conservation comme du phénol ou du chlorobutanol et des agents du même genre. La solution finie peut être stérilisée facilement par des techniques classiques de filtration.

La composition utilisée dans la présente invention contient en solution aqueuse une quantité suffisante de l'agent de l'invention pour être thérapeutiquement efficace. Les doses à administrer dépendent dans une large mesure de l'état du sujet que l'on traite et de son poids.

A titre d'exemple, on mentionnera une posologie de 100 à 5000 $\mu$g par injection, de préférence 500 $\mu$g par injection répétée trois fois par semaine. Ceci correspond à une dose approximative de 10 $\gamma$/kg chez l'homme.

Les produits activés de l'invention trouvent aussi une application à la préparation de traceurs radioactifs utilisables dans des procédés de dosage radioimmunologique pour doser le taux de FTS présent dans le sérum.

Le test des rosettes a permis de démontrer que le taux sérique de FTS est modifié dans certaines situations pathologiques immunitaires, telles que le lupus et les myasthénies (J. F. Bach et Coll., Ann. N.Y. Acad. Sci. *249* (1975) page 186).

Toutefois, étant donné que le test des rosettes est un essai semi-quantitatif, le besoin s'est fait sentir de mettre au point un procédé de dosage quantitatif, suffisamment sensible pour permettre un dosage direct du FTS dans le sang.

Le procédé de dosage du FTS dans un échantillon consiste à mettre en présence une quantitié déterminée de cet échantillon avec une quantité déterminée de traceur et une quantité déterminée d'anticorps anti-FTS de manière à provoquer une réaction de compétition entre le traceur et le FTS de l'échantillon dans leur liaison avec l'anticoprs anti-FTS, à déterminer après équilibre de la réaction la quantité de traceur marque, soit libre, soit lié à l'anticorps, et à comparer cette valeur à celles obtenues pour des quantitiés connues et croissantes de FTS.

L'anticorps anti-FTS est préparé en fixant le FTS ou l'un des ses analogues et dérivés sur un support pour obtenir un immunogène, en injectant l'immunogène à un animal et en recueillant ultérieurement le sérum de l'audinal.

Le traceur est obtenu par marquage par un isotope radioactif, de préférence en introduisant une molécule iodable sur un produit activé selon l'invention et en iodant ensuite le dérivé iodable ainsi obtenu.

Pour mettre en oeuvre le procédé de dosage on pourra prévoir un coffret de réactifs contenant essentiellement le traceur et l'anticorps nécessaires du dosage.

Le procédé de dosage est illustré par l'exemple suivant:

Exemple 4

I. Materiel et methodes

1. Réactifs

Le FTS de synthèse a été préparé selon la méthode indiquée plus haut.

2. Traceur radioactif

Un analogue synthétique du FTS, par exemple le P-amino benzoyl Glnl-FTS, préparé par E. Bricas (Université de Paris Sud Orsay) est marqué à l'Iode 125 en présence de chloramine T (le p-amino benzoyl Gln 1-FTS ne constitue qu'une exemple parmi d'autres des analogues ou dérivés du FTS pouvant être utilisés pour la préparation du traceur radioactif). Le FTS-$^{125}$I est séparé de l'iode libre par filtration sur gel de Sephadex G-25® élué en tampon Tris HCl 0,1M, pH 7,6. Des fractions aliquotes de FTS-$^{125}$I (environ $5 \times 10^6$ cpm) sont mélangées à une suspension de Chelex 100 (50 mg/ml) puis incubées 30 minutes en présence de 100 ng de chlorure de zinc. Le mélange est ensuite

12

chromatographié sur Biogel P2®, élué avec du tampon Tris HCl 0,1 M, pH 7,6 contenant 1% de gélatine. Le pic correspondant au FTS-Zn est récupéré et utilisé pour le dosage radioimmunologique.

### 3. Anticorps anti-FTS

Le FTS est rendu immunogénique en le couplant à la sérum albumine bovine en présence de glutaraldéhyde (6). L'immunogène ainsi obtenu est injecté, incorporé dans de l'adjuvant complet de Freund, à des lapins (1 mg per lapin). Une injection de rappel (également de 1 mg) est administrée un mois après la première injection et les ainmaux sont saignés 15 jours après l'injection de rappel.

### 4. Préparation des échantillons biologiques

Des sérums frais de souris normales et thymectomisées sont ultrafiltrés sur Amicon CF50® et utilisés extemporanément.

### II. Resultats

#### 1. Comparaison de l'antigénicité des deux traceurs

L'antisérum obtenu par immunisation contre le FTS de synthèse lie les deux traceurs étudiés (FTS $^{125}$I et FTS-Zn $^{125}$I). Défini comme la dilution maximale de l'antisérum donnant lieu à une liaison de 40% du traceur radioactif, le titre de l'antisérum est de 1/50 000 pour le FTS$^{125}$I et de 1/20 000 pour le FTS-Zn$^{125}$I.

#### 2. Déplacement de la liaison par le FTS et par le sérum de souris normale ou thymectomisée

Le FTS de synthèse inhibe la liaison des dux traceurs à l'anticorps, l'inhibition atteignant 80% aux plus fortes concentrations du peptide. La sensibilité du dosage exprimée par la dose d'hormone inhibant 50% de la liaison est de 10 pg pour le FTS$^{125}$I et 60—80 pg pour le FTS-Zn$^{125}$I.

Dix échantillons de sérum de souris normales et dix échantillons de sérums de souris thymectomisées ont été comparés pour leur capacité de déplacer la liaison des deux traceurs décrits plus haut à l'antisérum de lapin. Aucune différence significative n'est observée avec le FTS$^{125}$I entre les sérums provenant d'animaux normaux et thymectomisés. Par contre, alors que les dix échantillons de souris normales déplacent tous la liaison du FTS Zn$^{125}$I, aucun des sérums de souris thymectomisées (même utilisés non dilués) ne modifie la liaison. Le déplacement obtenu avec les sérums normaux (dilués au 1/2) varie de 40 à 60% selon les échantillons. Le déplacement est encore significatif quand les sérums sont dilués au 1/8ème.

### III. Discussion

Les résultats ci-dessus montrent que les anticorps obtenus chez le lapin après immunisation contre le FTS de synthèse lient à la fois les traceurs FTS et FTS-Zn, avec cependant un titre moins élevé pour FTS-Zn. Le FTS de synthèse déplace la liaison des deux traceurs à l'antisérum. Par contre, dans les conditions de ces expériences le FTS d'origine biologique, présent dans les sérums des souris normales, inhibe seulement la liaison du traceur FTS-Zn, la spécificité de l'inhibition étant démontrée par l'absence totale de déplacement obaservée avec les sérums de souris thymectomisées.

Ces résultats confirment l'existence de deux formes de FTS, l'une ayant capté un métal en l'occurrence le zonc, biologiquement active et antigéniquement proche de l'hormone naturelle, l'autre dépourvue de métal, biologiquement inactive et antigéniquement distincte de l'hormone thymique. Le FTS de synthèse, non passé sur Chelex 100®, qui est probablement biologiquement actif grâce aux traces de zinc qu'il contient (8) inclut à l'évidence, du FTS dépourvu de zinc. Son contenu en FTS-Zn ou en complexe de FTS avec d'autres métaux n'a pas été déterminé.

Il est encore difficile de dire si les lapins immunisés par le FTS de synthèse ont produit deux familles d'anticorps reconnaissant chacune une forme du peptide (avec ou sans zinc) ou plutôt essentiellement un anticorps donnant lieu à une réaction croisée contre le FTS et le FTS-Zn (mais reconnaissant le FTS avec une plus grande affinité que le FTS-Zn). Cette dernière hypothèse est compatible avec l'important (50%) déplacement de la liaison du traceur FTS-Zn observé à la fois avec le FTS de synthèse et le FTS naturel. Elle implique que le FTS naturel déplace seulement le traceur FTS-Zn dont il est plus proche structuralement que du FTS dépourvu de zinc et reste sans effet sur la liaison du traceur FTS dépourvu de zinc vis-à-vis duquel l'anticorps de lapin a une plus grande affinité.

**Revendications pour les Etats Contractants: BE, CH, LI, DE, FR, GB, IT, LU, NL, SE**

1. Procédé de préparation de nouveaux produits à partir du Facteur Thymique Sérique (FTS) ou de ses analogues ou dérivés, caractérisé par le fait que l'on triate le FTS ou l'un de ses analogues ou dérivés avec un métal ou une substance protéïque contenant un métal, ce métal étant susceptible de se lier au FTS ou son analogue ou dérivé et de former un complexe de coordination IV de configuration tétraédrique.

2. Procédé selon la revendication 1, caractérisé par le fait que l'on met en contact le FTS ou l'une de ses analogues ou dérivés avec un métal ou l'un de ses sels à un pH compris entre 7 et 9, pendant 10 à 30 minutes et à la température ambiante.

3. Procédé selon la revendication 2, caractérisé par le fait que l'on utilise une proportion de 1 mole de FTS ou d'analogue ou dérivé de FTS pour 0,1 à 10 moles de métal ou d'un de ses sels.

4. Procédé selon la revendication 2, caractérisé par le fait que le métal est un métal divalent ou un métal trivalent.

5. Procédé selon la revendication 4, caractérisé par le faite que le métal est le zinc.

6. Procédé selon la revendication 1, caractérisé par le fait que la substance protéïque contenant un métal est une fraction de sérum.

7. Procédé selon la revendication 1, caractérisé par le fait que la substance protéïque contenant un métal est une fraction d'un extrait thymique.

8. Procédé selon la revendication 7, caractérisé par le fait que la substance protéïque contenant un métal est une fraction de la Thymosine Fraction 5 dans une colonne de G-100 (Pharmacie 0,9 cm×100 cm) en tampon phosphate 0,2 M pH 7,3 afin d'éliminer la (les) molécule (s) semblable(s) au FTS, en conservant seulement les fractions éluées aved des volumes d'élution compris entre 15 et 22 ml.

9. Procédé selon la revendication 8, caractérisé par le fait que l'on fait incuber le FTS ou l'un de ses analogues et dérivés avec ladite fraction, on fractionne le mélange obtenu après incubation, et on sélectionne la fraction la plus active dans le test des rosettes.

10. Procédé selon la revendication 9, caractérisé par le fait que l'incubation est réalisée à un pH compris entre 6 et 8, pendant 10 minutes à 1 heure et à une température de 30 à 40°C.

11. Procédé selon la revendication 1, caractérisé par le fait que, préalablement au traitement avec la métal ou la substance protéïque contenant le metal, on traite le FTS ou l'une des ses analogues ou dérivés avec un agent de chélation capable de chélater les métaux.

12. Produit obtenu par la mise en oeuvre du procédé selon l'une des revendications 1 à 11.

13. A titre de produit nouveau, un produit caractérisé par le fait eque le FTS ou l'un des ses analogues ou dérivés est lié à un métal susceptible de former un complexe de coordination IV ayant une configuration tétraédrique.

14. Produit selon la revendication 13, caractérisé par le fait que le métal est un métal divalent ou trivalent.

15. Produit selon la revendication 14, caractérisé par le fait que le métal est le zinc.

16. Produit selon la revendication 13, caractérisé par le fait que le rapport en poids de métal au complexe est supérieur à 1/200.

17. A titre de médicament pour le traitement de maladies consécutives à un déséquilibre des lymphocytes T, un produit selon l'une des revendications 12 à 16.

18. Compsoition pharmaceutique contenant comme principe actif un produit selon l'une des revendications 12 à 16.

19. Traceur comprenant un produit selon l'une des revendications 12 à 16, marqué par un isotope radioactif.

20. Traceur selon la revendication 19, caractérisé par le fait que l'on introduit au préalable une molécule iodable sur le produit et que l'on iode ensuite le dérivé iodable ainsi obtenu.

21. Procédé de dosage du FTS contenu dans un échantillon, caractérisé par le fait que l'on met en présence une quantité déterminée dudit échantillon avec une quantité déterminée d'un traceur selon l'une des revendications 19 et 20 et une quantité déterminée d'anticorps anti-FTS de manière à provoquer une réaction de compétition entre le traceur et le FTS de l'échantillon dans leur liaison avec l'anticoprs anti-FTS, on détermine après équilibre de la réaction la quantité traceur , soit libre, sout lié à l'anticorps, et on compre cette valeur à celles obtenues pour des quantités connues et croissantes de FTS.

**Revendications pour l'Etat contractant: AT**

1. Procédé de préparation de nouveaux produits à partir du Facteur Thymique Sérique (FTS) ou de ses analogues ou dérivés, caractérisé par le fait que l'on traite le FTS ou l'un de ses analogues ou dérivés avec un métal ou une substance protéïque contenant un métal, ce métal étant susceptible de se lier au FTS ou son analogue ou dérivé et de former un complexe de coordination IV de configuration tétraédrique.

2. Procédé selon la revendication 1, caractérisé par le fait que l'on met en contact le FTS ou l'un de ses analogues ou dérivés avec un métal ou l'un de ses sels à un pH compris entre 7 et 9, pendant 10 à 30 minutes et à la température ambiante.

3. Procédé selon la revendication 2, caractérisé par le fait que l'on utilise une proportion de 1 mole de FTS ou d'analogue ou dérivé de FTS pour 0,1 à 10 moles de métal ou d'un de ses sels.

4. Procédé selon la revendication 2, caractérisé par le fait que le métal est un métal divalent ou un métal trivalent.

5. Procédé selon la revendication 4, caractérisé par le fait que le métal est le zinc.

6. Procédé selon la revendication 1, caractérisé par le fait que la substance protéïque contenant un métal est une fraction de sérum.

7. Procédé selon la revendication 1, caractérisé par le fait que la substance protéïque contenant un métal est une fraction d'un extrait thymique.

8. Procédé selon la revendication 7, caractérisé par le fait que la substance protéïque contenant un métal est une fraction de la Thymosine Fraction 5 dans une colonne de G-100 (Pharmacie 0,9 cm×100 cm) en tampon phosphate 0 2 M pH 7,3 afin d'éliminer la (les) molécule (s) semblable(s) au FTS, en conservant seulement les fractions éluées avec des volumes d'élution compris entre 15 et 22 ml.

9. Procédé selon la revendication 8, caractérisé par le fait que l'on fait incuber le FTS ou l'un de ses analogues et dérivés avec ladite fraction, on fractionne le mélante obtenu après incubation, et on sélectionne la fraction la plus active dans le test des rosettes.

10. Procédé selon la revendication 9, caractérisé par le fait que l'incubation est réalisée à un pH compris entre 6 et 8, pendant 10 minutes à 1 heure et à une température de 30 à 40°C.

11. Procédé selon la revendication 1, caractérisé par le fait que, préalablement au traitement avec le métal ou la substance protéïque contenant le métal, on traite le FTS ou l'un de ses analogues ou dérivés avec un agent de chélation capable de chélater les métaux.

12. Procédé selon l'une des revendications 1 à 11, caractérisé par le fait que l'on marque le produit du procédé par un isotope radioactif.

13. Procédé selon la revendication 12, caractérisé par le fait que l'on introduit au préalable une molécule iodable sur le produit et que l'on iode ensuite le dérivé iodable ainsi obtenu.

14. Procédé de dosage du FTS contenu dans un échantillon, caractérisé par le fait que l'on met en présence une quantité déterminée dudit échantillon avec une quantité déterminée d'un traceur selon l'une des revendications 12 et 13 et une quantité déterminée d'anticorps anti-FTS de manière à provoquer une réaction de compétition entre le traceur et le FTS de l'échantillon dans leur liaison avec l'anticorps anti-FTS, on détermine après équilibre de la réaction la quantité traceur, soit libre, soit lié à l'anticorps, et on compare cette valeur à celles obtenues pour des quantités connues et croissantes de FTS.

**Patentanspruche für die Vertragsstaaten: BE-CH-DE-FR-GB-IT-LI-LU-NL-SE**

1. Verfahren zur Herstellung von neuen Produkten aus serischem Thymusfaktor (STF) oder seinen Analoga oder Derivaten, dadurch gekennzeichnet, daß der STF oder eines seiner Analoga oder Derivate mit einem Metall oder einem metallhältigen Proteinstoff behandelt wird, wobei dieses Metall die Fähigkeit besitzt, sich mit dem STF oder seinem Analogon oder Derivat zu verbinden und einem Koordinationskomplex IV in Tetraederform zu bilden.

2. Verfahren nach Anspruch 1, dadurch gekennzeichnet, daß der STF oder eines seiner Analoga oder Derivate mit einem Metall oder einem seiner Salze bei einem pH-Wert zwischen 7 und 9, 10 bis 30 Minuten Lang bei Raumtemperatur in Berührung gebracht wird.

3. Verfahren nach Anspruch 2, dadurch gekennzeichnet, daß ein Verhältnis von 1 mol STF oder Analogon oder Derivat des STF zu 0,1 bis 10 mol Metall oder eines seiner Salze verwendet wird.

4. Verfahren nach Anspruch 2, dadurch gekennzeichnet, daß das Metall ein zweiwertiges oder ein dreiwertiges Metall ist.

5. Verfahren nach Anspruch 4, dadurch gekennzeichnet, daß das Metall Zink ist.

6. Verfahren nach Anspruch 1, dadurch gekennzeichnet, daß der metallhältige Proteinstoff eine Serumfraktion ist.

7. Verfahren nach Anspruch 1, dadurch gekennzeichnet, daß der metallhältige Proteinstoff eine Fraktion eines Thymusextraktes ist.

8. Verfahren nach Anspruch 7, dadurch gekennzeichnet, daß der metallhältige Proteinstoff eine Fraktion der Thymosinfraktion 5 in einer G-100 Säule (Pharmazie 0,9×100 cm) in Phosphatpuffer 0,2 M pH-Wert 7,3 ist, um das (die) dem STF ähnliche(n) Molekül(e) zu eliminieren, wobei nur die eluierten Fraktionen mit einem Eluierungsvolumen zwischen 15 und 22 ml konserviert werden.

9. Verfahren nach Anspruch 8, dadurch gekennzeichnet, daß der STF oder eines seiner Analoga oder Derivate mit der genannten Fraktion inkubiert wird, die erhaltene Mischung nach der Inkubation fraktioniert, und die aktivste Fraktion im Rossettentest selektiert wird.

10. Verfahren nach Anspruch 9, dadurch gekennzeichnet, daß die Inkubation bei einem pH-Wert zwischen 6 und 8, 10 Minuten bis 1 Stunde lang bei einer Temperatur zwischen 30 und 40°C durchgeführt wird.

11. Verfahren nach Anspruch 1, dadurch gekennzeichnet, daß vor der Behandlung mit dem Metall oder dem metallhältigen Proteinstoff der STF oder eines seiner Analoga oder Derivate mit einem Chelationsmittel behandelt wird, das die Fähigkeit besitzt, mit Metallen Chelate zu bilden.

12. Produkt erhalten unter Durchführung des Verfahrens nach einem der Ansprüche 1 bis 11.

13. Als neues Produkt, ein Produkt, dadurch gekennzeichnet, daß der STF oder eines seiner Analoga oder Derivate mit einem Metall verbunden ist, das die Fähigkeit besitzt, einen Koordinationskomplex IV mit Tetraederform zu bilden.

14. Produkt nach Anspruch 13, dadurch gekennzeichnet, daß das Metall ein zweiwertiges oder dreiwertiges Metall ist.

**0 041 019**

15. Produkt nach Anspruch 14, dadurch gekennzeichnet, daß das Metall Zink ist.

16. Produkt nach Anspruch 13, dadurch gekennzeichnet, daß das Gewichtsverhältnis zwischen Metall und Komplex größer ist als 1/200.

17. Als Medikament zur Behandlung von Krankheiten infolge eines gestörten Gleichgewichtes der T-Lymphozyten, ein Produkt nach einem der Ansprüche 12 bis 16.

18. Pharmazeutische Zusammensetzung, die als aktiven Bestandteil ein Produkt nach einem der Ansprüche 12 bis 16 enthält.

19. Tracer enthaltend ein Produkt nach einem der Ansprüche 12 bis 16, markiert mit Hilfe eines Radioisotopen.

20. Tracer nach Anspruch 19, dadurch gekennzeichnet, daß vorher ein jodierbares Molekül in das Produkt eingeführt wird und dann das so erhaltene jodierbare Derivat jodiert wird.

21. Verfahren zur Dosierung des in einer Probe enthaltenen STF, dadurch gekennzeichnet, daß eine bestimmte Menge der genannten Probe einer bestimmten Menge eines Tracers nach einem der Ansprüche 19 und 20 und einer bestimmten Menge anti-SFT Antikörper so gegenübergestellt wird, daß eine Wettlaufreaktion zwischen dem Tracer und dem STF der Probe in ihrer Verbindung mit dem anti-STF Antikörper hervorgerufen wird, dann nach erreichtem Reaktionsgleichgewicht die Tracermenge, entweder frei oder an den Antikörper gebunden, bestimmt und dieser Wert mit jenen verglichen wird, die für bekannte und wachsende Mengen von STF erhalten wurden.

**Patentansprüche für den Vertragsstaat: AT**

1. Verfahren zur Herstellung von neuen Produkten aus serischem Thymusfaktor (STF) oder seinen Analoga oder Derivaten, dadurch gekennzeichnet, daß der STF oder eines seiner Analoga oder Derivate mit einem Metall oder einem metallhältigen Proteinstoff behandelt wird, wobei dieses Metall die Fähigkeit besitzt, sich mit dem STF oder seinem Analogon oder Derivat zu verbinden und einen Koordinationskomplex IV in Tetraederform zu bilden.

2. Verfahren nach Anspruch 1, dadurch gekennzeichnet, daß der STF oder eines seiner Analoga oder Derivate mit einem Metall oder einem seiner Salze bei einem pH-Wert zwischen 7 und 9, 10 bis 30 minuten lang bei Raumtemperatur in Berührung gebracht wird.

3. Verfahren nach Anspruch 2, dadurch gekennzeichnet, daß ein Verhältnis von 1 mol STF oder Analogon oder Derivat des STF zu 0,1 bis 10 mol Metall oder eines seiner Salze verwendet wird.

4. Verfahren nach Anspruch 2, dadurch gekennzeichnet, daß das Metall ein zweiwertiges oder ein dreiwertiges Metall ist.

5. Verfahren nach Anspruch 4, dadurch gekennzeichnet, daß das Metall Zink ist.

6. Verfahren nach Anspruch 1, dadurch gekennzeichnet, daß der metallhältige Proteinstoff eine Serumfraktion ist.

7. Verfahren nach Anspruch 1, dadurch gekennzeichnet, daß der metallhältige Proteinstoff eine Fraktion eines Thymusextraktes ist.

8. Verfahren nach Anspruch 7, dadurch gekennzeichnet, daß der metallhältige Proteinstoff eine Fraktion der Thymosinfraktion 5 in einer G-100 Säule (Pharmazie 0,9×100 cm) in Phosphatpuffer 0,2 M pH-Wert 7,3 ist, um das (die) dem STF ähnliche(n) Molekül(e) zu eliminieren, wobei nur die eluierten Fraktionen mit einem Eluierungsvolumen zwischen 15 und 22 ml konserviert werden.

9. Verfahren nach Anspruch 8, dadurch gekennzeichnet, daß der STF oder eines seiner Analoga oder Derivate mit der genannten Fraktion inkubiert wird, die erhaltene Mischung nach der Inkubation fraktioniert, und die aktivste Fraktion im Rosettentest selektiert wird.

10. Verfahren nach Anspruch 9, dadurch gekennzeichnet, daß die Inkubation bei einem pH-Wert zwischen 6 und 8, 10 Minuten bis 1 Stunde lang bei einer Temperatur zwischen 30 und 40°C durchgeführt wird.

11. Verfahren nach Anspruch 1, dadurch gekennzeichnet, daß vor der Behandlung mit dem Metall oder dem metallhältigen Proteinstoff der STF oder eines seiner Analoga oder Derivate mit einem Chelationsmittel behandelt wird, das die Fähigkeit besitzt, mit Metallen Chelate zu bilden.

12. Verfahren nach einem der Ansprüche 1 bis 11, dadurch gekennzeichnet, daß das Verfahrensprodukt mit Hilfe eines Radioisotopen markiert wird.

13. Verfahren nach Anspruch 12, dadurch gekennzeichnet, daß vorher ein jodierbares Molekül in das Produkt eingeführt wird und dann das so erhaltene jodierbare Derivat jodiert wird.

14. Verfahren zur Dosierung des in einer Probe enthaltenen STF, dadurch gekennzeichnet, daß eine bestimmte Menge der genannten Probe einer bestimmten Menge eines Tracers nach einem der Ansprüche 12 und 13 und einer bestimmten Menge anti-STF Antikörper so gegenübergestellt wird, daß eine Wettlaufreaktion zwischen dem Tracer und dem STF der Probe in ihrer Verbindung mit dem anti-STF Antikörper hervorgerufen wird, dann nach erreichtem Reaktionsgleichgewicht die Tracermenge, entweder frei oder an den Antikörper gebunden, bestimmt und dieser Wert mit jenen verglichen wird, die für bekannte und wachsende Mengen von STF erhalten wurde.

16

**0 041 019**

## Claims for the Contracting States: BE-CH-DE-FR-GB-IT-LI-LU-NL-SE

1. A process for the production of new products using Thymic Seric Factor (TSF) or the analogs or derivatives thereof, characterised in that TSF or one of the analogs or derivatives thereof is treated with a metal or a protein substance containing a metal, the said metal being capable of combining with TSF or the analog or derivative thereof and forming a coordination complex IV of tetrahedral configuration.

2. A process according to claim 1, characterised in that TSF or an analog or derivative thereof is brought into contact with the metal or salt thereof at a pH of from 7 to 9 for from 10 to 30 minutes at ambient temperature.

3. A process according to claim 2, characterised in that a proportion of 1 mol of TSF or an analog or derivative of TSF is used for from 0.1 to 10 mols of metal or a salt thereof.

4. A process according to claim 2, characterised in that the metal is a divalent or trivalent metal.

5. A process according to claim 4, characterised in that the metal is zinc.

6. A process according to claim 1, characterised in that the protein substance containing a metal is a serum fraction.

7. A process according to claim 1, characterised in that the protein substance containing a metal is a fraction of a thymic extract.

8. A process according to claim 7, characterised in that protein substance containing a metal is a fraction of the Thymosine Fraction 5 using a G-100 column (Pharmacy 0.9 cm×100 cm) and a 0.2 M phosphate buffer of pH 7.3 to remove the molecule(s) which are similar to TSF and retaining only the eluted fractions with elution volumes between 15 and 22 ml.

9. A process according to claim 8, characterised in that the TSF or the analog or derivative thereof is incubated with the said fraction, the mixture which is obtained after incubation is fractionated and the fraction which is most active in the rosette test is selected.

10. A process according to claim 9, characterised in that incubation is carried out at a pH of from 6 to 8 over a period of from 10 minutes to 1 hour and at a temperature of from 30 to 40°C.

11. A process according to claim 1, characterised in that the TSF or one of the analogs or derivatives thereof is treated with a chelating agent which is capable of chelating the metals before the treatment with the metal or the protein substance containing the metal.

12. A product which is obtained by carrying out the process according to one of claims 1 to 11.

13. A product, as a new product, characterised in that TSF or an analog or derivative thereof is joined to a metal which is capable of forming a coordination complex IV having a tetrahedral configuration.

14. A product according to claim 13, characterised in that the metal is a divalent or trivalent metal.

15. A product according to claim 14, characterised in that the metal is zinc.

16. A product according to claim 13, characterised in that the ratio, by weight, of metal to the complex is greater than 1/200.

17. A product according to one of claims 12 to 16 as a medicament for treating conditions which result from an imbalance of the T lymphocytes.

18. A pharmaceutical composition containing as active principle a product according to one of claims 12 to 16.

19. A tracer consisting of a product according to one of claims 12 to 16, marked by a radioactive isotope.

20. A tracer according to claim 19, characterised in that an iodizable molecule is previously introduced onto the product and the iodizable derivative which is thus obtained is subsequently iodized.

21. A process for assaying the TSF which is contained in a sample, characterised in that a predetermined quantity of the said sample is brought together with a predetermined quantity of a tracer according to one of claims 19 and 20 and with a predetermined quantity of TSF-antibodies to produce in completition a reaction between the tracer and the TSF of the sample when they come into contact with the TSF-antibody, after the reaction has equilibrated the quantity of tracer is determined, which is either free or linked to the anti-body and this value is compared with the values obtained for known and increasing quantities of TSF.

## Claims for the Contracting State: AT

1. A process for the production of new products using Thymic Seric Factor (TSF) or the analogs or derivatives thereof, characterised in that TSF or one of the analogs or derivatives thereof is treated with a metal or a protein substance containing a metal, the said metal being capable of combining with TSF or the analog or derivative thereof and forming a coordination complex IV of tetrahedral configuration.

2. A process according to claim 1, characterised in that TSF or an analog or derivative thereof is brought into contact with the metal or salt thereof at a pH of from 7 to 9 for from 10 to 30 minutes at ambient temperature.

3. A process according to claim 2, characterised in that a proportion of 1 mol of TSF or an analog or derivative of TSF is used for from 0.1 to 10 mols of metal or a salt thereof.

17

**0 041 019**

4. A process according to claim 2, characterised in that the metal is a divalent or trivalent metal.

5. A process according to claim 4, characterised in that the metal is zinc.

6. A process according to claim 1, characterised in that the protein substance containing a metal is a serum fraction.

7. A process according to claim 1, characterised in that the protein substance containing a metal is a fraction of a thymic extract.

8. A process according to claim 7, characterised in that protein substance containing a metal is a fraction of the Thymosine Fraction 5 using a G-100 column (Pharmacy 0.9 cm×100 cm) and a 0.2 M phosphate buffer of pH 7.3 to remove the molecule(s) which are similar to TSF and retaining only the eluted fractions with elution volumes between 15 and 22 ml.

9. A process according to claim 8, characterised in that the TSF or the analog or derivative thereof is incubated with the said fraction, the mixture which is obtained after incubation is fractionated and the fraction which is most active in the rosette test is selected.

10. A process according to claim 9, characterised in that incubation is carried out at a pH of from 6 to 8 over a period of from 10 minutes to 1 hour and at a temperature of from 30 to 40°C.

11. A process according to claim 1, characterised in that the TSF or one of the analogs or derivatives thereof is treated with a chelating agent which is capable of chelating the metals before the treatment with the metal or the protein substance containing the metal.

12. A process according to one of claims 1 to 11, characterised in that the process product is marked by a radioactive isotope.

13. A process according to claim 12, characterised in that an iodizable molecule is previously introduced onto the product and the iodizable derivative which is thus obtained is subsequently iodized.

14. A process for assaying the TSF which is contained in a sample, characterised in that a predetermined quantity of the said sample is brought together with a predetermined quantity of a tracer according to one of claims 12 and 13 and with a predetermined quantity of TSF-antibodies to produce in competition a reaction between the tracer and the TSF of the sample when they come into contact with the TSF-antibody, after the reaction has equilibrated the quantity of tracer is determined, which is either free or linked to the antibody and this value is compared with the values obtained for known and increasing quantities of TSF.

FIG_1

0 041 019

FIG. 2

FIG_3

FIG_4

DILUTION DANS
LE SERUM

$\frac{1}{4.10^6}$

$\frac{1}{1.10^6}$

$\frac{1}{256000}$

$\frac{1}{64000}$

$\frac{1}{16000}$

$\frac{1}{4000}$

$\frac{1}{1000}$

$\frac{1}{256}$

Ia

Ih

Ij

Ii

II

30'    45'    1H    1H30    TEMPS

0 041 019

FIG_5

FIG_6